# FASCICULE DE BREVET EUROPEEN

(11) **EP 1 272 229 B1**
(45) Date de publication et mention de la délivrance du brevet: **19.11.2003**
(21) Numéro de dépôt: 01917194.1
(22) Date de dépôt: 21.03.2001
(51) Int. Cl.: A61L 15/46, A61L 15/48, A61L 15/28, A61L 15/26

(54) **COMPRESSE ANTISEPTIQUE**
ANTISEPTISCHE KOMPRESSE
ANTISEPTIC COMPRESS

(30) Priorité: 22.03.2000 FR 0003665
(43) Date de publication de la demande: 08.01.2003
(73) Titulaire: LABORATOIRES D'HYGIENE ET DE DIETETIQUE, 21300 Chenove (FR)
(72) Inventeur: APERT, Laurent, F-21000 Dijon (FR); AUGUSTE, Stéphane, F-21490 VAROIS ET CHAIGNOT (FR)
(74) Mandataire: Hubert, Philippe
(86) Numéro de dépôt international: FR0100834
(87) Numéro de publication internationale: WO01070285

(56) Documents cités:
- FR-A- 2 753 380
- FR-A- 2 775 903
- GB-A- 1 599 159
- GB-A- 2 098 074
- US-A- 6 039 940

## Description

La présente invention concerne une compresse antiseptique destinée à favoriser la cicatrisation des plaies infectées ou à risque d'infection, ainsi qu'un pansement incorporant la compresse antiseptique.

### Art antérieur

On sait déjà que des pansements capables de maintenir un certain degré d'humidité à la surface d'une plaie ont une action favorable au processus de cicatrisation. Cette propriété est utilisée dans de nombreux pansements, tels que par exemple "ALGOPLAQUE" commercialisé par les Laboratoires URGO ou "COMFEEL" commercialisé par la société COLOPLAST, qui utilisent une dispersion d'hydrocolloïdes dans une matrice hydrophobe adhésive ; dans ces pansements, les particules d'hydrocolloïdes absorbent les exsudats et maintiennent un environnement humide favorable à la cicatrisation. Toutefois, dans le cas de plaies fortement exsudatives, l'absorption est insuffisante et on peut observer une accumulation de liquides qui risquent de devenir rapidement un foyer d'infection.

Un autre type de pansement, connu sous la dénomination tulle gras, utilise des graisses et des huiles telles que la vaseline officinale et la paraffine pour former une protection de la plaie. Dans ce cas, l'absorption des exsudats n'est pas possible et il faut prévoir une compresse dans lé cas des plaies suintantes. Dans les autres cas, la plaie a tendance à sécher et on observe alors fréquemment des adhérences entre le pansement et les tissus fraîchement régénérés. Il s'ensuit un renouvellement douloureux du pansement, quand ce n'est pas une destruction des tissus cicatrisés. Pour éviter les problèmes d'infection dans les plaies, il est connu d'ajouter au pansement des agents antiseptiques destinés à limiter ou mieux réduire le développement des agents pathogènes infectieux. Dans ce domaine, on connaît par exemple des pansements tels que UNITUL commercialisé par la société BAMA-GEVE qui contiennent des agents antiseptiques dispersés dans une pommade à base de corps gras hydrophobes. On connaît également, selon EP-A-689425 un gel hydratant comprenant un système hydrocolloïde à base d'alginate et de carboxyméthylcellulose sodique et un système de conservateur constitué par des agents antimicrobiens et des agents antifongiques. Il s'agit ici d'un hydrogel contenant une forte proportion d'eau (97 %) dans lequel les agents actifs sont en solution. On connaît également, selon une formulation proche, le pansement de marque CONNETIVINA PLUS, commercialisé par la société FIDIA, qui est constitué d'un hydrogel à base de polyéthylèneglycol et qui contient de la sulfadiazine argentique en dispersion et un sel de l'acide hyaluronique.

On connaît aussi FR 2 775 903 qui décrit une masse adhésive hydrocolloïde pour pansement, dont le pouvoir absorbant est amplifié et accéléré en raison de la présence combinée d'un hydrocolloïde et d'un agent tensioactif. Cette masse adhésive peut accessoirement contenir un agent antiseptique qui pourra être facilement diffusé lorsque le pansement aura absorbé un fluide aqueux capable de dissoudre en partie ledit agent antiseptique.

Dans le cas des pansements comportant une couche de contact hydrophobe, notamment à base de corps gras ou d'élastomères, il est beaucoup plus difficile d'obtenir une action antiseptique au moyen de principes actifs dispersés dans la couche de contact. Ces principes actifs se trouvent emprisonnés dans la structure hydrophobe et présentent de ce fait une biodisponibilité extrêmement faible. Ce problème est encore plus prononcé lorsque l'on utilise des structures de pansement peu ou non absorbants, comprenant des élastomères assez cohésifs dans le but d'éviter la destructuration du pansement au moment du retrait de la plaie. Pour cette raison, il n'existe pas actuellement de pansement contenant à la fois des élastomères de synthèse et des agents antiseptiques dans une couche de contact non adhésive et non absorbante.

### Objet de l'invention

La présente invention vise à fournir une compresse de contact non adhésive et peu absorbante pour des plaies ou des brûlures de la peau qui présentent un risque infectieux, ladite compresse étant à base de corps gras et d'hydrocolloïdes, ainsi qu'un pansement incorporant une telle compresse.

### Description

Selon l'invention, on a trouvé qu'il était possible d'obtenir une compresse constituée d'une couche de contact cohésive amphiphile non adhésive et présentant une action antiseptique. On a en effet trouvé que la biodisponibilité des agents antiseptiques dispersés dans la couche de contact pouvait être considérablement améliorée en présence d'un agent tensioactif non ionique dont la balance hydrophile/lipophile (HLB) est supérieure à 10.

La compresse selon l'invention, constituée d'une structure cohésive à base d'élastomères de synthèse du type tribloc fortement plastifiée par une huile non polaire est caractérisée en ce qu' elle contient en dispersion une faible quantité d'hydrocolloïdes, au moins un agent antiseptique et au moins un agent tensioactif de balance hydrophile/lipophile (HLB) supérieure à 10.

Selon un aspect particulier de l'invention, les élastomères de synthèse sont des élastomères tribloc à séquence centrale saturée du type copolymère de blocs polystyrène et de blocs polyéthylène-butylène (SEBS) ou copolymère de blocs polystyrène et de blocs polyéthylène-propylène (SEPS).

Selon un autre aspect particulier de l'invention, l'huile non polaire est une huile de paraffine, une vaseline officinale ou un mélange de ces composés.

Selon un autre aspect particulier de l'invention, les hydrocolloïdes sont présents sous forme de carboxyméthylcellulose (CMC) sodique.

Selon un autre aspect particulier de l'invention, le tensioactif est du type non-ionique et choisi parmi les dérivés polyéthoxylés des esters du sorbitol avec un acide gras.

Selon un autre aspect particulier de l'invention, les agents antiseptiques sont des principes actifs anti-microbiens et/ou des principes actifs antifongiques, de préférence choisis parmi les sels d'argent.

Selon un mode préféré de réalisation de l'invention, la couche de contact avec la plaie est discontinue et de préférence constituée d'un tissu à mailles ouvertes, totalement enrobé d'un gel cohésif et non adhérent de façon à laisser les mailles essentiellement non obturées, ledit gel étant formé d'une matrice élastomérique hydrophobe fortement plastifiée et contenant une dispersion d'hydrocolloïdes, au moins un agent antiseptique et au moins un agent tensioactif dont la balance hydrophile/lipophile (HLB) est supérieure à 10.

### Description détaillée

La compresse antiseptique selon l'invention comprend une couche de contact avec la plaie essentiellement constituée d'un gel cohésif et non adhérent dans lequel sont dispersés des hydrocolloïdes, un ou plusieurs agents antiseptiques et un agent tensioactif de balance hydrophile/lipophile (HLB) supérieure à 10.

Le gel cohésif, qui a pour fonction de constituer la structure de la couche de contact et d'assurer le caractère non adhérent du pansement. est constitué d'une matrice élastomérique de préférence à base d'éléments triblocs, associée à une quantité importante d'une huile ou corps gras non polaire qui va jouer à la fois le rôle de plastifiant pour la matière élastomérique et un rôle antiadhérent. On obtient ainsi un gel cohésif non adhésif et très élastique, très conformable et très résistant à la déchirure.

Les élastomères utilisés préférentiellement sont du type SEBS ou SEPS à poids moléculaire élevé, tels que ceux commercialisés par exemple par la société SHELL sous la référence Kraton G 1651 ou par la société KURARAY sous la dénomination SEPTON. Ces élastomères sont présents dans la couche de contact à raison d'environ 3 à 8 % en poids.

Le choix particulier de ces élastomères triblocs permet d'obtenir un gel dépourvu de pouvoir adhésif, parfaitement antiadhérent vis à vis des tissus régénérés et de la peau périlésionnelle.

L'huile non polaire est généralement une huile ou un mélange d'huiles minérale(s) à base d'hydrocarbures, qui peut être fluide ou plus ou moins épaisse.

Dans le cadre de l'invention, on utilisera préférentiellement une huile de paraffine, une vaseline officinale ou un mélange de ces composés.

De préférence encore, on utilisera une ou plusieurs huiles minérales commercialisées par la société SHELL sous la dénomination ONDINA, préférentiellement la référence ONDINA 15 seule(s) ou avantageusement en mélange avec une vaseline officinale conforme à la pharmacopée française.

L'huile non polaire est présente à raison d'environ 55 à 90 % du poids de la couche de contact.

La compresse comprend également en dispersion dans la matrice élastomérique, une faible quantité d'hydrocolloïdes parmi lesquels on préfère la carboxyméthylcellulose (CMC) sodique, dont la fonction est de maintenir un environnement humide favorable au processus de cicatrisation. Dans le cas de l'utilisation de la CMC sodique, celle-ci est présente sous forme d'une poudre fine, de granulométrie voisine de 50 à 100 µm, à raison de 3 à 20 % préférentiellement 4 à 14 %, environ du poids de la couche de contact. Cette quantité d'hydrocolloïde est faible mais suffisante pour maintenir un degré d'humidité de la surface en contact avec la plaie sans conférer un caractère très absorbant à la compresse et permet d'éviter un gonflement de la couche de contact.

Notamment, les compresses selon l'invention présentent un pouvoir absorbant négligeable ou très faible vis à vis des fluides aqueux et leur mise en contact avec de l'eau n'entraîne qu'une augmentation de poids minime généralement inférieure à 50 % et préférentiellement inférieure à 25 % du poids de la couche de contact.

D'autres hydrocolloïdes, comme par exemple les alginates peuvent être utilisés en lieu et place de la CMC. dans le cadre de l'invention.

La couche de contact contient également des agents antiseptiques en dispersion dans la matière élastomérique. On choisit des principes actifs connus pour leurs propriétés antimicrobiennes ou antifongiques, notamment des composés actifs pour lutter contre le développement des germes pathogènes tels que par exemple *staphylococcus aureus* (staphylocoque doré) ou *pseudomonas aeruginosa* (pyocyanique).

Parmi ces principes actifs, utilisables seuls ou en combinaison, on préfère la chlorhexidine, l'hexamidine, la bétadine ou des sels d'argent tels que par exemple la sulfadiazine argentique, le chlorure d'argent ou le nitrate d'argent. La quantité présente dans la couche de contact dépend de la nature du principe actif et varie entre 0,2 et 5 %.

Selon l'invention, on ajoute au moins un tensioactif sans lequel l'activité de l'agent antiseptique ne serait pas obtenue correctement. On choisit un agent tensioactif non ionique dont la balance hydrophile/lipophile (HLB) est supérieure à 10 et, de préférence supérieure à 14, c'est-à-dire présentant un caractère à prédominance nettement hydrophile. Cet agent tensioactif doit être présent de préférence à raison de 1 à 6 % en poids dans la formulation. Parmi les produits disponibles dans le commerce et convenant pour réaliser l'invention, on préfère par exemple les esters de sorbitol polyéthoxylés, notamment le polysorbate 80 disponible sous la référence Montanox 80 auprès de la société SEPPIC, qui présente une balance hydrophile/lipophile (HLB) d'environ 15 à 16.

De façon classique, la formulation comprend également des additifs destinés à assurer la stabilité du produit, tels que par exemple des antioxydants ou des agents protecteurs de la lumière.

La masse élastomérique constituant la couche de contact de la compresse est réalisée suivant un procédé hot-melt par malaxage à chaud des différents constituants, l'hydrocolloïde et le principe actif étant ajoutés après obtention d'un mélange homogène des élastomères et du plastifiant huileux. Le mélange obtenu est ensuite enduit sur un support souple et conformable qui peut être par exemple un film, un non tissé ou un tissu pour obtenir un pansement applicable directement sur la peau.

Selon l'un des modes de réalisation préférés de l'invention, le mélange fondu peut être aussi enduit sur un tissu à mailles larges de façon à enrober totalement les fils constituant le tissu et laisser les ouvertures de mailles non obturées. Le tissu utilisé pour réaliser cette compresse nouvelle est préférentiellement composé de fils synthétiques à fibres longues. On obtient ainsi une couche discontinue très conformable renforcée par le filet, qui associe des propriétés favorables à la cicatrisation à la propriété antiseptique.

Cette compresse antiseptique, qui s'utilise de façon analogue à un tulle gras, peut être mise en contact direct avec une plaie ou une brûlure, recouverte par un tampon absorbant et maintenue par une bande ou un adhésif.

A titre d'exemple on a réalisé une compresse (E1) à partir d'une masse élastomérique préparée selon le procédé hot-melt à partir de 1,52 kg d'huile de paraffine (obtenue auprès de la société SHELL sous la référence ONDINA 15), 100 g d'élastomère SEBS à haut poids moléculaire (Kraton G 1651 commercialisé par la société SHELL), 100 g de vaseline officinale, 2,5 g d'antioxydant phénolique, 300 g de carboxyméthylcellulose sodique en poudre fine (ref 7H4XF commercialisée par la société AQUALON), 100 g de polysorbate 80 (commercialisé sous la référence MONTANOX 80 par la société SEPPIC) et 60 g de sulfadiazine argentique en poudre fine, (de qualité conforme à l'USP). La masse en fusion a été enduite sur un tissu à mailles ouvertes du type marquisette en fils de polyester 33 dtex, présentant des mailles carrées dont l'ouverture est d'environ 1mm². Le tissu est enrobé par passage dans un bain de la masse élastomérique fondu à 135-145°C puis l'excédent du gel est éliminé par laminage entre deux cylindres. La bande du tissu enrobé est refroidie rapidement par un flux d'air froid. La quantité de gel déposée sur les fils est d'environ 145 g/m² ce qui correspond à environ 4 g/m² de sulfadiazine argentique. La bande obtenue est ensuite complexée avec un film protecteur en polyester opaque sur chacune de ses faces, puis découpée en fragments de dimension convenable pour une utilisation sous forme de compresse et enfin conditionnée en sachet étanche.

Le caractère antiseptique de la compresse (E1) selon l'invention réalisée ci-dessus, a été testé in vitro par contact avec un milieu de culture. Pour cette étude, on a utilisé en comparaison :
a) une compresse témoin (A) de formulation analogue à la compresse (E1) ci-dessus, mais ne contenant ni principe actif, ni tensioactif
b) une compresse (B) de formulation analogue à la compresse (E1) contenant le principe actif (soit 2,75 % de sulfadiazine argentique), mais pas de tensioactif
c) une compresse (C) de type analogue à celle décrite au témoin (A) c'est-à-dire sans principe actif ni tensioactif, sur laquelle on a déposé une couche de gel de Flammazine de 3 mm, suivant une pratique couramment appliquée dans les hôpitaux. La Flammazine est une crème émulsionnée contenant 1 % de sulfadiazine argentique.

Les essais ont été conduits sur deux souches bactériennes : *staphylococcus aureus* et *pseudomonas aeruginosa* qui sont fréquemment responsables d'infections nocosomiales et/ou de surinfections chez les grands brûlés. Des disques de 10 mm de diamètre sont découpés dans chacune des compresses à tester et déposés sur des géloses inoculées dans la masse ou en surface par une suspension bactérienne de titre connu. Après incubation pendant 24 heures à 37°C, les diamètres des zones d'inhibition de croissance bactérienne sont mesurés et comparés.

Pour cette expérimentation, on a utilisé des géloses trypcase-soja (G1) ou Mueller-Hinton 2 (G2) (fournies par la société BIOMERIEUX) qui ont été inoculées dans la masse des géloses par 1 ml d'une suspension bactérienne titrant environ 10⁹ ou 10⁶ UFC/ml pour 15 à 18 ml de gélose, ou en surface par 1 ml d'une suspension bactérienne titrant environ 10⁸ UFC/ml. L'unité UFC est une unité formant une colonie ou "Unit Forming Cell" en anglais. Les résultats obtenus, représentés par le diamètre des zones d'inhibition exprimé en mm, sont reportés dans les tableaux 1 à 3.

**TABLEAU 1**

| *Pseudomonas aeruginosa* 10⁹ UFC/ml | | | | |
|---|---|---|---|---|
| Compresse | G 1 | | G2 | |
| | masse | surface | masse | surface |
| A | 10 | 10 | 10 | 10 |
| B | 10 | 10 | 10,5 | 10,5 |
| C | 13 | 12 | 12 | 11 |
| E1 | 15 | 13 | 15 | 14 |

**TABLEAU 2**

| *Staphylococcus aureus* 1,2.10⁹ UFC/ml | | | | |
|---|---|---|---|---|
| Compresse | G1 | | G2 | |
| | masse | surface | masse | surface |
| A | 10 | 10 | 10 | 10 |
| B | 10 | 11 | 10 | 10 |
| C | 12 | 12 | 11 | 10 |
| E1 | 11 | 14 | 12 | 10.5 |

**TABLEAU 3**

| Gélose Mueller Hinton 2 inoculée dans la masse | | |
|---|---|---|
| | *Pseudomonas aeruginosa* 2.9.10⁶ UFC/ml | *Staphylococcus Aureus* 2.1.10⁸ UFC/ml |
| A | 10 | 10 |
| B | 10,5 | 11,5 |
| C | 17 | 16,5 |
| E1 | 17 | 16 |

Dans les tableaux ci-dessus, un résultat 10 signifie qu'aucune zone autour du disque de la compresse de diamètre 10 mm n'est exempte de croissance bactérienne.

Les résultats supérieurs à 10 signifient qu'une zone périphérique autour de la compresse est exempte de croissance bactérienne.

Le tableau 1 concerne l'inhibition de croissance de *Pseudomonas aeruginosa* sur les deux types de gélose, inoculées en masse ou en surface à une concentration de l'ordre de 10⁹ UFC/ml. Le tableau 2 concerne le même type d'expérience conduite avec *staphylococcus aureus.* Le tableau 3 est relatif à l'inhibition de la croissance des deux souches bactériennes à des concentrations de l'ordre de 10⁶ UFC/ml.

Les résultats obtenus montrent une totale inactivité, comme attendu, de la compresse dépourvue de principe actif (A), une activité antiseptique très faible de la compresse contenant le principe actif mais dépourvue de tensioactif, et une bonne inhibition de la croissance bactérienne en présence de la compresse conforme à l'invention (E1), l'inhibition obtenue étant généralement au moins équivalente à celle obtenue en présence de la compresse associée à une couche épaisse de Flammazine. Les résultats confirment également la nécessité de la présence d'un agent tensio-actif de type hydrophile pour obtenir une biodisponibilité de l'agent antiseptique au niveau de la surface à aseptiser.

Le pouvoir absorbant d'une couche de contact réalisée avec la masse élastomérique selon l'exemple précédent a été mesuré, en comparaison avec une masse adhésive hydrocolloïde telle que décrite dans le document FR 2775903. Pour cet essai. on a réalisé une couche continue du mélange formulé selon l'exemple E1 de la présente invention avec un grammage d'environ 600 g /m². L'exemple comparatif correspond à la masse adhésive absorbante conformément à l'exemple 1 du document FR 2775903, avec un grammage de 350 g/m². Pour effectuer la mesure du pouvoir absorbant, on a immergé un échantillon de 25 cm² de chacun des produits pendant une heure dans une solution de sérum physiologique maintenue à 37 °C. A l'issue du temps d'immersion, les échantillons ont été égouttés et pesés. Les augmentations de poids calculées à partir de ces résultats montrent un pouvoir absorbant de 144 g/m² pour l'exemple selon la présente invention et de 1575 g/m² pour l'exemple comparatif. Exprimés en pourcentage pondéral, les augmentations de poids sont respectivement de 144/600 = 24 % pour l'exemple selon l'invention et 1575/350 = 450 % pour l'exemple comparatif.

Les pansements obtenus en utilisant une compresse selon l'invention présentent ainsi de nombreux avantages pour le traitement des plaies ou des brûlures accompagnées d'un risque infectieux : leur composition permet d'obtenir à la fois un contact gras non adhérent par la présence d'une huile ou d'une vaseline, un environnement humide par la présence d'un hydrocolloïde et un milieu aseptisant créé par une diffusion suffisante d'un agent antiseptique. Par ailleurs, la structure de la couche élastomérique qui constitue avec les autres ingrédients la couche de contact permet une manipulation facile et un retrait doux et indolore du pansement et ne laissant pas de matière sur les tissus nouvellement régénérés et provenant de la couche de contact.

## Revendications

1. Compresse pour le soin des plaies ou brûlures, dont la surface de contact avec la plaie ou la brûlure est constituée d'une matrice élastomérique plastifiée par une huile non polaire, ladite matrice élastomérique contenant en dispersion un hydrocolloïde, **caractérisé en ce que** ladite matrice élastomérique contient également en dispersion au moins un agent antiseptique et au moins un tensioactif dont la balance hydrophile/lipophile (HLB) est supérieure à 10, ladite huile non polaire représentant 55 à 90 % du poids de la compresse.

2. Compresse selon la revendication 1, **caractérisée en ce que** la structure de la matrice élastomérique est constituée par un élastomère de synthèse de type tribloc, préférentiellement du type tribloc à séquence centrale saturée choisi parmi les copolymères de blocs polystyrène et de blocs polyéthylène-butylène (SEBS) ou les copolymères de blocs polystyrène et de blocs polyéthylène-propylène (SEPS) de haut poids moléculaire.

3. Compresse selon l'une des revendications 1 ou 2, **caractérisée en ce que** l'huile non polaire est choisie parmi les huiles de paraffine, la vaseline officinale ou les mélanges de ces composés.

4. Compresse selon l'une des revendications 1 à 3, **caractérisée en ce que** l'hydrocolloïde est la carboxyméthylcellulose sodique.

5. Compresse selon l'une des revendications 1 à 4, **caractérisée en ce que** le tensio-actif est du type non ionique et choisi parmi les dérivés polyéthoxylés des esters du sorbitol avec un acide gras.

6. Compresse selon l'une des revendications 1 à 5, **caractérisée en ce que** l'agent antiseptique est un sel d'argent.

7. Compresse selon l'une quelconque des revendications 1 à 6, **caractérisée en ce que** la surface de contact avec la plaie est celle d'une couche discontinue enduite sur un support tissé à mailles larges de façon à enrober totalement les fils du tissu et laisser les ouvertures de maille essentiellement non obturées.

8. Pansement obtenu avec une compresse selon l'une quelconque des revendications 1 à 7, **caractérisé en ce que** ladite compresse est disposée sur un support constitué par un film ou un non tissé souple susceptible d'être adhésivé.

## Patentansprüche

1. Kompresse zur Behandlung von Wunden oder Verbrennungen, deren Berührungsfläche mit der Wunde oder der Verbrennung durch eine elastomere Matrix gebildet wird die durch ein unpolares Öl weich gemacht wird, wobei die elastomere Matrix eine Hydrocolloid in Form einer Dispersion enthält, **dadurch gekennzeichnet, dass** die elastomere Matrix, gleichfalls als Dispersion, mindestens ein antiseptisches Mittel und mindestens ein oberflächenaktives Mittel enthält dessen hydrophil-lipophiles Gleichgewicht höher als 10 ist, wobei das unpolare Öl 55-90 Gew.-% der Kompresse darstellt.

2. Kompresse gemäß Anspruch 1, **dadurch gekennzeichnet dass** die Struktur der elastomeren Matrix durch ein synthetisches Dreiblock-Elastomer gebildet wird, vorzugsweise vom Dreiblock-Typ mit einer zentralen gesättigten Sequenz, ausgewählt aus Copolymeren mit Polystyrolblocks und Polyethylenbutylenblocks (SEBS) oder Copolymeren mit Polystyrolblocks und Polyethylenpropylenblocks (SEPS) mit hohem Molekulargewicht.

3. Kompresse gemäß einem der Ansprüche 1 oder 2, **dadurch gekennzeichnet dass** das unpolare Öl ausgewählt ist aus Paraffinölen, offizineller Vaseline oder Gemischen dieser Verbindungen.

4. Kompresse gemäß einem der Ansprüche 1 bis 3, **dadurch gekennzeichnet dass** das Hydrocolloid Natriumcarboxymethylcellulose ist.

5. Kompresse gemäß einem der Ansprüche 1 bis 4, **dadurch gekennzeichnet dass** das oberflächenaktive Mittel nicht ionisch ist und ausgewählt ist aus polyethoxylierten Derivaten von Sorbitestern mit einer Fettsäure.

6. Kompresse gemäß einem der Ansprüche 1 bis 5, **dadurch gekennzeichnet dass** das antiseptische Mittel ein Silbersalz ist.

7. Kompresse gemäß einem der Ansprüche 1 bis 6, **dadurch gekennzeichnet dass** die Berührungsfläche mit der Wunde diejenige einer unterbrochenen Schicht ist die auf einen großmaschig gewobenen Träger in einer Art und Weise aufgebracht ist dass die Fäden des Gewebes vollständig umhüllt sind und dass die Maschenöffnungen im Wesentlichen ungefüllt bleiben.

8. Verband erhalten mit einer Kompresse gemäß einem der Ansprüche 1 bis 7, **dadurch gekennzeichnet dass** die Kompresse auf einen Träger aufgebracht ist der durch eine flexible Folie oder ein flexibles Vlies gebildet wird die klebefähig gemacht werden können.

## Claims

1. Compress for the treatment of wounds or burns, whose contact surface with the wound or burn consists of an elastomeric matrix plasticized with a non-polar oil, said elastomeric matrix containing a hydrocolloid in dispersion, **characterized in that** said elastomeric matrix also contains, in dispersion, at least one antiseptic and at least one surfactant with a hydrophilic/lipophilic balance (HLB) greater than 10, said non-polar oil representing 55 to 90% of the weight of the compress.

2. Compress according to claim 1, **characterized in that** the structure of the elastomeric matrix consists of a synthetic elastomer of the tri-block type, preferably of the tri-block type with a saturated central block, selected from high molecular copolymers of polystyrene blocks and polyethylene-butylene blocks (SEBS) and high molecular weight copolymers of polystyrene blocks and polyethylene-propylene blocks (SEPS).

3. Compress according to claim 1 or 2, **characterized in that** the non-polar oil is selected from paraffin oils, officinal petrolatum and mixtures of these compounds.

4. Compress according to one of claims 1 to 3, **characterized in that** the hydrocolloid is sodium carboxymethyl cellulose.

5. Compress according to one of claims 1 to 4, **characterized in that** the surfactant is of the non-ionic type and is selected from polyethoxylated derivatives of sorbitol fatty acid esters.

6. Compress according to one of claims 1 to 5, **characterized in that** the antiseptic is a silver salt.

7. Compress according to any one of claims 1 to 6, **characterized in that** the surface in contact with the wound is in the form of a discontinuous layer which has been coated onto a wide-mesh woven substrate in such a way as to coat the threads of the fabric totally and leave the mesh apertures essentially unobstructed.

8. Dressing obtained with a compress according to any one of claims 1 to 7, **characterized in that** said compress is placed on a substrate consisting of a film or a flexible nonwoven fabric capable of being rendered adhesive.
